# EUROPEAN PATENT APPLICATION

(11) **EP 4 592 403 A1**
(43) Date of publication of application: **30.07.2025**
(21) Application number: 22966090.7
(22) Date of filing: 22.11.2022
(51) Int. Cl.: C12Q 1/6869, C12M 1/34

(54) **GENE SEQUENCING CHIP, PACKAGE STRUCTURE, SYSTEM, AND CLEAN, PRODUCTION AND SEQUENCING METHODS**

(71) Applicant: MGI Tech Co., Ltd., Shenzhen, Guangdong 518083 (CN)
(72) Inventor: HUANG, Fuxing, Shenzhen, Guangdong 518083 (CN); GENG, Weijie, Shenzhen, Guangdong 518083 (CN); SHI, Xing, Shenzhen, Guangdong 518083 (CN)
(74) Representative: Meissner Bolte Nürnberg
(86) International application number: PCT/CN2022/133536
(87) International publication number: WO 2024/108390

(57) **Abstract**

A gene sequencing chip and a package structure thereof, a cleaning method, a production method, and a system. The gene sequencing chip comprises at least one chip body. The chip body comprises: a substrate layer provided with a circuit; at least one electrode layer located on the substrate layer; and at least one protective layer located on the substrate layer and used for keeping the insulativity between the substrate layer and the outside. The protective layer and the electrode layer are not located on a same plane, at least part of the electrode layer is exposed out of the protective layer, and the portion of the electrode layer exposed out of the protective layer comprises a plurality of electrode parts, wherein the plurality of electrode parts are arranged in an array, and DNBs are loaded to the electrode parts to form a sequencing area. Instead of using existing design of molecular hierarchical modification, the gene sequencing chip can be produced using only a conventional semiconductor manufacturing process and on the basis of a conventional semiconductor material; in cooperation with a protective layer, arrayed sites formed by the electrode layer are resistant to corrosion of a biochemical reagent, the structure is more suitable for adsorption and binding of DNA, and the morphology and material still keep original characteristics after repeated use; and the gene sequencing chip can be repeatedly used.

## Description

### TECHNICAL FIELD

The present disclosure relates to the field of semiconductor chips related to gene sequencing, and in particular to a gene sequencing chip, a packaging structure, a system and method for cleaning and manufacturing thereof, and a sequencing method using the same.

### BACKGROUND OF THE INVENTION

The reduction in sequencing cost has brought rapid advances in gene technology, such as increasing the number of test sites per unit area, reducing the volume of reagents used, increasing the flow rate of reagents within the chip to reduce sequencing time, and the like, as proposed in the prior art.

A large part of the sequencing cost comes from the manufacture and design of gene sequencing chip. The next-generation gene sequencing microarray chip in the prior art utilizes different characteristics of surface monomolecular layer to allow for differential molecular layer modification on the chip wafer surface, *i.e.,* to immobilize DNA after PCR specifically at required sites, for the purpose of, on the chip surface, forming amination sites which can specifically adsorb DNA and a region without specific adsorbability to the DNA, which allows to form a regular arrayed or random pattern to capture the gene sequences to be sequenced at specific adsorption sites.

However, this type of chip has the adsorbed DNA and various residual proteins and biochemical reagents on surface, which cannot be effectively removed after sequencing is completed. Alternatively, the monomolecular layer modification on the surface of a gene sequencing chip may be damaged during cleaning of the sequencing chip for reuse, rendering the chip unable to regularly capture DNA to be sequenced.

The above properties make the next-generation gene sequencing chip a "disposable consumable", which greatly increases the cost of single sequencing and makes the entry barrier of sequencing higher.

### SUMMARY OF THE INVENTION

The present disclosure is proposed in consideration of the above. The present disclosure is intended to provide a new chip structure and a corresponding packaging structure and system, as well as a method for fabricating and cleaning the chip structure and a sequencing method by applying the chip structure, for application in the field of gene sequencing. It is expected to reduce cost and improve efficiency while taking into account stability and reliability.

In order to solve some of the above technical problems, in a first aspect, the present disclosure provides a gene sequencing chip, comprising at least one chip body, the chip body comprising a substrate layer provided with a circuit; at least one electrode layer on the substrate layer; and at least one protective layer on the substrate layer, for insulation of the substrate layer from the outside, wherein the protective layer and the electrode layer are not located on the same plane, at least a portion of the electrode layer protrudes from the protective layer, and the portion of the electrode layer protruding from the protective layer comprises a plurality of electrode parts presenting an array arrangement and loaded with DNA Nanoballs (DNBs) to form a sequencing region.

In a further embodiment of the present disclosure, the electrode layer is formed with a transition metal oxide, wherein the transition metal oxide comprises at least one of titanium dioxide, zirconium dioxide, tantalum pentoxide, niobium hexoxide and hafnium dioxide.

In an optional embodiment of the present disclosure, the protective layer is disposed on the substrate layer, the electrode layer fully protrudes from the protective layer, and the electrode layer has a plurality of electrode parts presenting an array arrangement disposed on the protective layer.

In an optional embodiment of the present disclosure, the protective layer comprises a first protective layer and a second protective layer, and the first protective layer, the electrode layer and the second protective layer are sequentially laminated on the substrate layer; the electrode layer is disposed on the first protective layer; and the second protective layer is provided with a plurality of second openings presenting an array arrangement and exposing the corresponding portions of the electrode layer to the corresponding second openings, to form a plurality of electrode parts on the electrode layer.

In an optional embodiment of the present disclosure, the electrode layer is disposed on the substrate layer; and the protective layer is disposed on the electrode layer and provided with a plurality of first openings presenting an array arrangement and exposing the corresponding portions of the electrode layer to the corresponding first openings, to form a plurality of electrode parts on the electrode layer.

In a further embodiment of the present disclosure, the gene sequencing chip comprises two chip bodies connected by splicing along the lengthwise direction.

In a further embodiment of the present disclosure, there are two gene sequencing chips, and the two gene sequencing chips are stacked along the thickness direction, and arranged with the sides provided with the electrode layers being opposite.

In a second aspect, the present disclosure further provides a packaging structure for a gene sequencing chip, comprising the gene sequencing chip as described above and a packaging body encasing the gene sequencing chip to form an open structure or a microfluidic structure.

In a third aspect, the present disclosure further provides a method for manufacturing a gene sequencing chip, comprising steps of: S1, providing at least one substrate layer; S2, disposing at least one electrode layer and at least one protective layer on the substrate layer with the protective layer and the electrode layer being not on the same plane; and S3, etching on the electrode layer or windowing the protective layer, so that at least a portion of the electrode layer protrudes from the protective layer to form a plurality of electrode parts presenting an array arrangement.

In a further embodiment of the present disclosure, the electrode layer is composed of a transition metal oxide comprising at least one of titanium dioxide, zirconium dioxide, tantalum pentoxide, niobium hexaoxide and hafnium dioxide.

In an optional embodiment of the present disclosure, step S2 comprises disposing the protective layer on the substrate layer and disposing the electrode layer on the protective layer; and step S2 comprises etching on the electrode layer to form a plurality of electrode parts protruding from the protective layer on the electrode layer.

In an optional embodiment of the present disclosure, step S2 comprises disposing a first protective layer on the substrate layer; disposing the electrode layer on the first protective layer; and disposing a second protective layer on the electrode layer; and step S3 comprises windowing the second protective layer to form a plurality of second openings presenting an array arrangement and exposing the corresponding portions of the electrode layer to the corresponding second openings, so as to form a plurality of electrode parts on the electrode layer.

In an optional embodiment of the present disclosure, step S2 comprises disposing the electrode layer on the substrate layer and disposing the protective layer on the electrode layer; and step S3 comprises windowing the protective layer to form a plurality of first openings presenting an array arrangement and exposing the corresponding portions of the electrode layer to the corresponding first openings, so as to form a plurality of electrode parts on the electrode layer.

In an optional embodiment of the present disclosure, there are two gene sequencing chips, and the method for manufacturing further comprises S4, stacking and packaging the two gene sequencing chips with the sides provided with electrode layers being opposite.

In addition, the present disclosure further provides a system for gene sequencing, comprising the gene sequencing chip as described above; a test reagent container for holding reaction reagents; a gripping mechanism for picking up the gene sequencing chip and putting it into the test reagent container for biochemical reactions; and a signal acquisition mechanism for acquiring signals from the gene sequencing chip to realize sequencing.

In a further embodiment of the present disclosure, the test reagent container is provided with a plurality of reagent chambers for holding different reaction reagents.

In a further embodiment of the present disclosure, the gripping mechanism comprises a support portion below the gene sequencing chip, and a handle portion connected to the support portion and protruding above the gene sequencing chip.

In an optional embodiment of the present disclosure, the gripping mechanism has an L-shaped handle structure.

Finally, embodiments of the present disclosure provide a method for cleaning a gene sequencing chip as follows:
allowing a first cleaning solution to flow through the gene sequencing chip or placing the gene sequencing chip in a first cleaning solution for a first predetermined period; cleaning the chip surface repeatedly with deionized water; and placing the gene sequencing chip in a stream of inert gas for complete drying, wherein the first cleaning solution is a potassium hydroxide solution containing a nonionic surfactant;
   or
allowing a second cleaning solution heated to a first predetermined temperature flow through the gene sequencing chip or placing the gene sequencing chip in a second cleaning solution heated to a first predetermined temperature for a second predetermined period; cleaning the chip surface repeatedly with deionized water; and placing the gene sequencing chip in a stream of inert gas for complete drying, wherein the second cleaning solution is a mixture of ammonia water and hydrogen peroxide in a predetermined ratio;
   or
pre-treating the gene sequencing chip to expose the gene sequencing chip; placing the chip in a plasma atmosphere for a third predetermined period and then cleaning the chip surface repeatedly with deionized water; and placing the gene sequencing chip in a stream of inert gas for complete drying;
   or
heating and sonication of the gene sequencing chip directly exposed to isopropanol for a fourth predetermined period, and then cleaning the chip surface repeatedly with deionized water; rinsing or immersing the gene sequencing chip with sodium dodecyl sulfate solution, and cleaning the chip surface again repeatedly with deionized water; and placing the gene sequencing chip in a stream of inert gas for complete drying.

In addition, the present disclosure further provides a method for sequencing utilizing the gene sequencing chip as described above, comprising preparing DNBs; loading the DNBs onto the gene sequencing chip as described above; and sequencing the DNBs loaded onto the gene sequencing chip.

In a further embodiment of the present disclosure, the gene sequencing chip is a gene sequencing chip capable of being reused after cleaning.

In a further embodiment of the present disclosure, the cleaning is performed using the method as described above.

In a further embodiment of the present disclosure, the sequencing is automated sequencing or manual sequencing.

In a further embodiment of the present disclosure, the efficiency of loading the DNBs is determined.

In summary, the present disclosure proposes a gene sequencing chip, wherein an electrode layer and a protective layer are disposed on a substrate layer, and the electrode layer is structurally designed to present an arrayed site arrangement. In contrast to the existing designs using molecular layer modifications, it has following advantages:
1. The chip structure may be realized by applying conventional semiconductor manufacturing processes and conventional semiconductor materials, without special materials and processes, such as special silicon wafer substrates. The process has been well-established and has high yield, and can reduce the cost during the manufacturing.
2. With a protective layer, the arrayed sites formed on the electrode layer are resistant to the corrosion of biochemical reagents, and the structure is more suitable for DNA adsorption and binding. In addition, the chip may still possess the original characteristics in terms of the morphology and material after some times of reuse, and thus may be reused.
3. In the prior art, the monomolecular layer modification is not resistant to high temperature, sensitive to pH and special components of the reagents, unstable in performance, prone to failure of the specific adsorption layer in a variety of common conditions and even after long storage, and prone to physical contact damage (such as surface rubbing, etc.), all of which may result in a decrease in the output throughput. The chip structure of the present disclosure is more reliable compared to those in the prior art.
4. In the prior art, a chip has a molecular modification layer and a physically planar surface, the nucleic acids adsorbed on the surface are easily washed away in the high-speed flow of reagents, resulting in interrupted sequencing. In contrast, the chip structure provided in the embodiment of the present disclosure has a protruding surface, which may better retain the nucleic acids and improve the stability of sequencing.

### BRIEF DESCRIPTION OF THE DRAWINGS

In order to illustrate the specific embodiments of the present disclosure or the technical solutions in the prior art more clearly, the accompanying drawings to be used in the description of the specific embodiments or prior art will be briefly described below. Obviously, the drawings in the following description are some of the embodiments of the present disclosure, and other drawings may be obtained in accordance with these drawings without inventive efforts for those skilled in the art.
FIG. 1 illustrates a schematic diagram showing the structure of a gene sequencing chip exemplified in an example of the present disclosure;
FIG. 2 illustrates a wafer cross-sectional view of a chip structure having arrayed protruding sites exemplified in Example 1 of the present disclosure;
FIG. 3 illustrates a wafer cross-sectional view of a chip structure having grid-like sites exemplified in Example 2 of the present disclosure;
FIG. 4 illustrates a wafer cross-sectional view of a chip structure having recessed arrayed sites exemplified in Example 3 of the present disclosure;
FIG. 5 illustrates a schematic diagram of the structure obtained by stacking and packaging the chips provided in Example 1;
FIG. 6 illustrates a schematic diagram of the structure obtained by stacking and packaging the chips provided in Example 2;
FIG. 7 illustrates a schematic diagram of the structure obtained by stacking and packaging the chips provided in Example 3;
FIG. 8 illustrates a cross-sectional view of a packaging structure of a gene sequencing chip provided in an example of the present disclosure;
FIG. 9 illustrates a top view of a packaging structure of a gene sequencing chip provided in an example of the present disclosure;
FIG. 10 illustrates a cross-sectional view of a gene sequencing chip and a gripping mechanism in a fixed state provided in an example of the present disclosure;
FIG. 11 illustrates a structural schematic diagram of a system for gene sequencing provided in an example of the present disclosure;
FIG. 12 illustrates an optical signal graph of a gene sequencing chip provided in an example of the present disclosure for the first loading of DNBs;
FIG. 13 illustrates an optical signal graph of a gene sequencing chip provided in an example of the present disclosure after 6 times of cleanings for the loading of DNBs ;
FIG. 14 illustrates the comparison of sequencing quality of a gene sequencing chip provided in an example of the present disclosure at cycle 1, cycle 6 and cycle 150;
FIG. 15 illustrates AFM plots of the gene sequencing chips in an example of the present disclosure before and after immersion in different sequencing reagents for 5 days;
FIG. 16 illustrates the comparison of the sequencing signals in the first use and the reuse of the chip in an example of the present disclosure; and
FIG. 17 illustrates a cross-sectional and top view of a manual loading device in an example of the present disclosure, respectively.

**Reference signs in drawings**

| | | | |
|---|---|---|---|
| 100, | Gene sequencing chip; | 200, | Packaging body; |
| 300, | Packaging structure; | 400, | System; |
| 101, | Substrate layer; | 102, | Electrode layer; |
| 103, | Protective layer; | a, | Electrode part; |
| A, | First opening; | B, | Second opening; |
| 401, | Test reagent container; | 402, | Gripping mechanism; |
| 403, | Signal acquisition mechanism; | 402a, | Support portion; |
| 402b, | Handle portion; | 10, | Chip body; |
| 201, | Circuit pin; | | |
| 1-1, | Silicon-based chip; | 1-2, | Padding device; |
| 1-3, | Flowing reagent and a cavity thereof; | 1-4, | Upper cover plate. |

### DETAILED DESCRIPTION OF THE INVENTION

In order to make the foregoing, as well as other features and advantages of the present disclosure clearer, the disclosure is further described below in conjunction with the accompanying drawings. It should be understood that the specific embodiments given herein are for the purpose of explanation to those skilled in the art and are exemplary and not limiting.

In the description of the present disclosure, it should be understood that the terms "center", "longitudinal", "lateral", "length", "width", "thickness", "up", "down", "front", "back", "left", "right", "vertical", "horizontal", "top", "bottom", "inside", "outside", "clockwise", "counterclockwise", "axial", "radial", and "circumferential", etc. refer to an orientation or a position based on that shown in the drawings. These terms are intended only to facilitate description of the present disclosure and to simplify the description, are not intended to indicate or imply that the device or element as referred to must be in a particular orientation, or be constructed and operated in a particular orientation, and therefore cannot be construed as a limitation of the present disclosure.

Furthermore, the terms "first" and "second" are used for descriptive purposes only and are not to be understood as indicating or implying relative importance or as implicitly specifying the quantity of technical features indicated. Thus, a feature defined with the terms "first" or "second" may explicitly or implicitly include at least one such feature. In the description of the present disclosure, the term "a plurality of" means at least two, such as two, three, etc., unless otherwise expressly and specifically defined.

In the present disclosure, unless otherwise expressly specified or defined, the terms "installation", "linkage", "connection", and "fixation", etc. should be understood in a broad sense, such as, a fixed connection, a removable connection, or an integral connection; a mechanical connection, or an electrical connection; a direct connection, or an indirect connection through an intermediary medium, such as a connection within two elements, or an interaction between two elements, unless otherwise expressly defined. Those skilled in the art may understand the specific meanings of the above terms in the present disclosure on a case-by-case basis.

In the present disclosure, unless otherwise expressly specified and defined, a first feature being "above" or "below" a second feature may indicate that the first feature has direct contact, or indirect contact through an intermediate medium, with the second feature. Furthermore, a first feature being "above", "over" and "on" a second feature may indicate that the first feature is directly above or obliquely above the second feature, or may simply indicate that the first feature is horizontally above the second feature. A first feature being "below", "under" and "underneath" a second feature may indicate that the first feature is directly below or obliquely below the second feature, or may simply indicate that the first feature is horizontally below the second feature.

Following the above, the present disclosure proposes a plurality of solutions based on a general inventive concept in response to the technical problems existing in the prior art, aiming at solving the cost and other technical problems brought about by the non-reusability due to the molecular layer chip structure currently adopted.

### [General Inventive Concept]

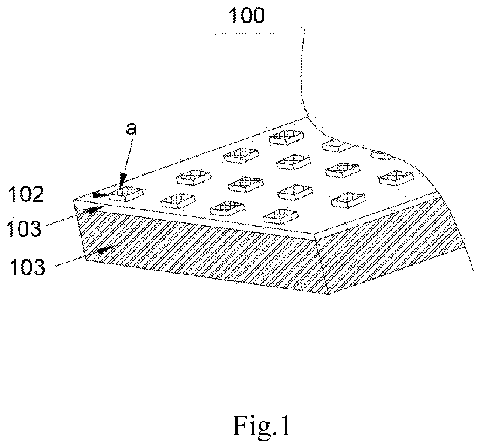

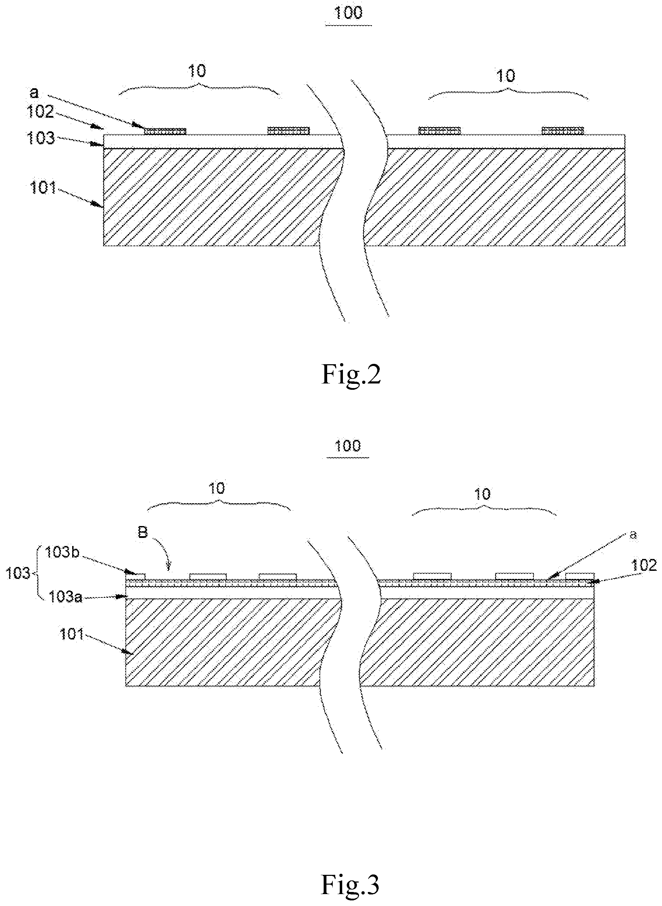

Firstly, with reference to FIG. 1 and FIG. 2, FIG. 1 illustrates a schematic diagram showing the structure of a gene sequencing chip exemplified in an example of the present disclosure, and FIG. 2 illustrates a wafer cross-sectional view of a chip structure having arrayed protruding sites exemplified in an example of the present disclosure. The gene sequencing chip 100 comprises a plurality of chip bodies 10;
the chip body 10 comprises a substrate layer 101, an electrode layer 102, and a protective layer 103;
the substrate layer 101 is used to provide a carrier for the chip, is located at a relatively bottom layer of the chip, and may be provided with internal circuits to realize corresponding functional effects; and
further, the substrate layer 101 may optionally be any one of a wafer composed of quartz, glass or silicon and an integrated circuit wafer.

The electrode layer 102 and the protective layer 103 are disposed on the surface of the substrate layer 101. The electrode layer 102 is used for contacting with the liquid to be detected to trigger electrical signals so as to realize sequencing. The protective layer 103 is used for insulation of the substrate layer 101from the outside to avoid the liquid to be detected from penetrating into the substrate layer 101 and damaging the circuit components of the chip.

In embodiments of the present disclosure, the electrode layer 102 is composed of a transition metal oxide selected from at least one of titanium dioxide, zirconium dioxide, tantalum pentoxide, niobium hexoxide, and hafnium dioxide.

The protective layer 103 is composed of silicon oxide or silicon nitride, or may be other insulating films.

In the arrangements employed in the general inventive concept, the protective layer 103 and the electrode layer 102 are not located on the same plane, at least a portion of the electrode layer 102 protrudes from the protective layer 103, and the portion of the electrode layer 102 protruding from the protective layer 103 comprises a plurality of "electrode parts a" raised or recessed relative to the substrate layer 101 and presenting an array arrangement.

In the arrangements, the relative position of the electrode layer 102 and the protective layer 103 may be exchanged, *i.e.,* the electrode layer 102 may be located above or below the protective layer 103, provided that the "electrode parts a" of the electrode layer 102 present an array arrangement.

The pattern arrangements and quantity of the arrayed sites constituted by the "electrode parts a" may be designed adaptively according to the desired requirements. For example, the arrayed pattern may be constituted by the sites that protrude from the surface or are in the grid-like structures, or grooves recessed into the wafer surface.

The "electrode parts a" presenting the arrayed sites are correspondingly loaded with DNBs (DNA Nanoballs) to form a sequencing region, and the sequencing may be realized by contacting the sequencing region with the liquid to be detected, the liquid reacting with different reagents, and subsequently capturing optical signals with different wavelengths and energies fed back from the DNBs on the sequencing region followed by decoding. In the present disclosure, the DNA nanoballs refer to DNA nanoballs obtained by rolling circle amplification of circular DNA molecules and subsequently loaded for sequencing. The core of this technology is that a genomic DNA fragment is cyclized into a circular single-stranded DNA, and then a single-stranded DNA is formed from multiple copies of the circular single-stranded DNAs connected end to end by rolling circle amplification (RCA) technology, and is freely folded into a nanoball structure, *i.e.,* DNA nanoball (DNB), in a solution. The main process of DNB paired-end sequencing comprises the following steps: firstly fragmenting the genomic DNA, adding an adapter sequence, and cyclizing the fragments to form a circular single-stranded DNA; then amplifying the circular single-stranded DNA by 2-3 orders of magnitude by using rolling circle amplification technology, so as to obtain amplification products called DNA nanoballs; and ultimately immobilizing the DNA nanoballs on an arrayed silicon chip by a DNB loading technology, followed by: (a) performing the combinatorial probe-anchor synthesis (cPAS); (b) sequencing a forward strand; (c) forming a reverse strand by a high-fidelity polymerase with a chain displacement function; and (d) sequencing the reverse strand by DNA molecular anchor. The process has the advantages of fast synthesis, high accuracy, etc.

It may be understood that the sites for connecting the DNBs as referred to in the embodiments of the present disclosure, *i.e.,* the electrode parts constituted by the electrode layer 102, are connected to the DNBs by electrostatic adsorption force. In the solution, the electrode parts are positively charged, and the DNBs are negatively charged, resulting in attraction between them.

The solutions proposed by the general inventive concept are described by specific examples below.

### [Chip Structure]

### Example 1

Continuing to refer to FIG. 2.

In an example of the present disclosure, the gene sequencing chip 100 comprises a plurality of chip bodies 10 comprising a substrate layer 101, an electrode layer 102 and a protective layer 103.

The substrate layer 101 is a semiconductor wafer, the material of which may be selected from any one of a quartz wafer, a glass wafer, a silicon-based wafer and an integrated circuit wafer. The protective layer 103 is composed of silicon oxide or silicon nitride. The electrode layer 102 is composed of a transition metal oxide.

It may be understood that the chip body 10 is processed with a whole piece of semiconductor wafer, and a whole chip body 10 is formed by slicing.

As to the structural relationship, the protective layer 103 is disposed on the substrate layer 101, and then the electrode layer 102 is disposed on the protective layer 103 and forms a sequencing region together with the DNBs, resulting in a layered structure from bottom to top constituted by the substrate layer 101 - the protective layer 103 - the electrode layer 102, wherein the electrode layer 102 is exposed on the protective layer 103. The electrode layer 102 is designed such that a plurality of "electrode portions a" are arranged in an array on the protective layer 103, *i.e.,* forming arrayed sites raised relative to the protective layer 103 as the highest surface.

In this example, there may be no limitation on the morphology of the chip body 10, which may have a round or square overall structure and electrode layer 102.

### Example 2

With reference to FIG. 3, FIG. 3 illustrates a wafer cross-sectional view of a chip structure having grid-like sites exemplified in Example 2 of the present disclosure. The gene sequencing chip 100 comprises a plurality of chip bodies 10, each of which comprises a substrate layer 101, an electrode layer 102 and a protective layer 103 in the example of the present disclosure.

As above, the substrate layer 101 is a semiconductor wafer, the material of which may be selected from any one of a quartz wafer, a glass wafer, a silicon-based wafer, or an integrated circuit wafer. The protective layer 103 is composed of silicon oxide or silicon nitride. The electrode layer 102 is composed of a transition metal oxide.

Further, in terms of the structure, the protective layer 103 comprises a first protective layer 103a and a second protective layer 103b, wherein the first protective layer 103a is disposed on the substrate layer 101; the electrode layer 102 is disposed on the first protective layer 103a; the second protective layer 103b is disposed on the electrode layer 102, and the second protective layer 103b is designed to comprise second openings (windows) B presenting an array arrangement and exposing the corresponding portions of the covered electrode layer 102, thereby forming a plurality of "electrode parts a" with the second protective layer 103b. Accordingly, the "electrode parts a" present grid-like arrayed sites relative to the protective layer 103.

In this example, there may be no limitation on the morphology of the chip body 10. The chip body 10 and the electrode layer 102 may be round or square.

### Example 3

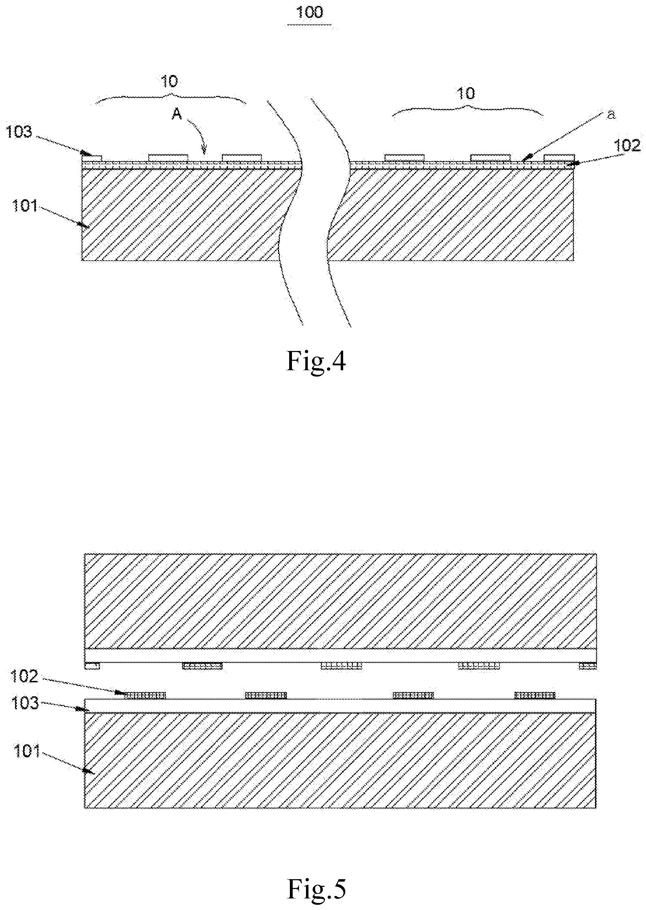

With reference to FIG. 4, FIG. 4 illustrates a wafer cross-sectional view of a chip structure having recessed arrayed sites exemplified in Example 3 of the present disclosure. The gene sequencing chip 100 comprises a plurality of chip bodies 10, each of which comprises a substrate layer 101, an electrode layer 102 and a protective layer 103 in the example of the present disclosure.

As above, the substrate layer 101 is a semiconductor wafer, the material of which may be selected from any one of a quartz wafer, a glass wafer, a silicon-based wafer, or an integrated circuit wafer. The protective layer 103 is composed of silicon oxide or silicon nitride. The electrode layer 102 is composed of a transition metal oxide.

In terms of the structure, the electrode layer 102 is disposed on the substrate layer 101; and the protective layer 103 is disposed on the electrode layer 102 and is provided with a plurality of first openings A presenting an array arrangement and exposing the corresponding portions of the electrode layer 102 to the corresponding first openings A, to form a plurality of "electrode parts a" on the electrode layer 102. Accordingly, the "electrode parts a" form recessed arrayed sites relative to the protective layer 103 as the highest surface.

### Example 4

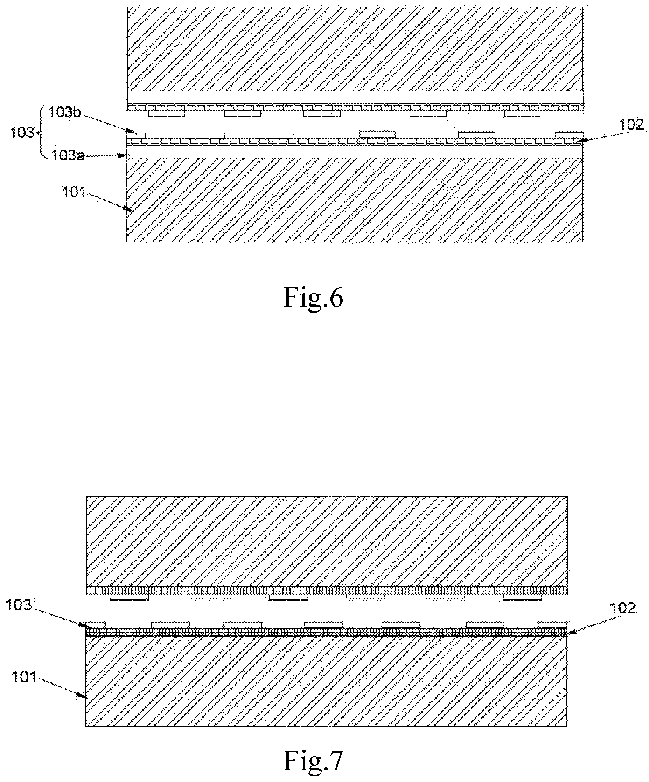

With reference to FIG. 5 to FIG. 7, FIG. 5 to FIG. 7 illustrate a stacked packaged chip structure exemplified in Example 4 of the present disclosure, wherein FIG. 5 illustrates a schematic diagram of the structure obtained by stacking and packaging the chips provided in Example 1; FIG. 6 illustrates a schematic diagram of the structure obtained by stacking and packaging the chips provided in Example 2; and FIG. 7 illustrates a schematic diagram of the structure obtained by stacking and packaging the chips provided in Example 3.

That is, a stacked packaged chip body 10 is formed by stacking the chip structures exemplified in Examples 1-3 above.

As shown in FIG. 5, two chip bodies 10 of Example 1 are stacked opposite each other along the thickness direction, and a gap is retained between the 2 electrode layers 102 presenting arrayed raised sites to facilitate the contact of reagents with the DNBs loaded on the sequencing region for biochemical reactions.

The substrate layer 101 is a semiconductor wafer, the material of which may be selected from any one of a quartz wafer, a glass wafer, a silicon-based wafer, or an integrated circuit wafer. The protective layer 103 is composed of silicon oxide or silicon nitride. The electrode layer 102 is composed of a transition metal oxide.

Continuing to refer to FIG. 6 and FIG. 7, as above, two identical test chips provided in Example 2 and Example 3 are used respectively, and are likewise stacked along the thickness direction, with the sides provided with the electrode layers 102 being opposite.

Further, the electrode layers 102 with the arrayed raised sites are designed to be staggered in the stacking and packaging to allow the contacting of the sequencing region more even, and the width of the channels between the electrode layers 102 is maintained to allow for better reagent inflowing.

It may be understood that the amount of reagents used may be further reduced by stacking and packaging the gene sequencing chips 100, integrating and packaging multiple units, etc., such as packaging the above-described two gene sequencing chips 100 opposite each other. Such a structure allows for multicore construction of a three-dimensional structure of the sequencing chip with a higher throughput and reduces the overall volume of the sequencing chip, therefore providing the advantages of compactness, portability, fast detection and low cost.

### [Packaging Structure]

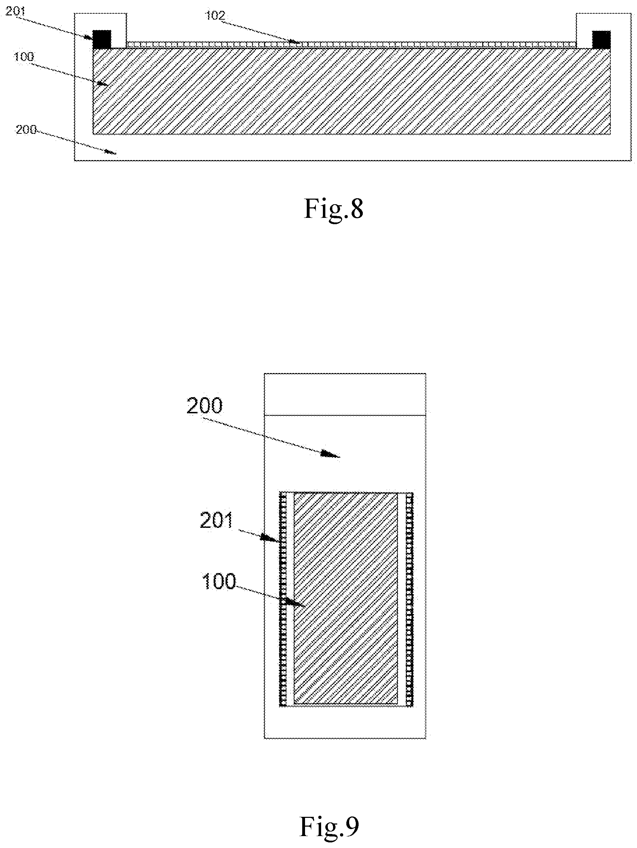

With reference to FIG. 8 and FIG. 9, FIG. 8 illustrates a cross-sectional view of a packaging structure 300 of a gene sequencing chip 100 provided in an example of the present disclosure; and FIG. 9 illustrates a top view of the packaging structure 300 provided in an example of the present disclosure.

Further, an example of the present disclosure provides a packaging structure 300 for gene sequencing, comprising:
the gene sequencing chip 100 as provided in any one of the above Examples 1-4 in the "[Chip Structure]" section; and
a packaging body 200, wrapping around the chip body 10 to form an open structure or a microfluidic structure.

It may be understood that the packaging body 200 is a fluid medium when not being molded, and is molded into a packaging structure with a certain external shape by a transfer molding method, *i.e.,* a fluid medium such as epoxy molding material is extruded into a mould cavity embedded on a substrate used for packaging and is subjected to curing and molding after embedding the chip body 10 therein. The packaging body 200 may protect the chip body 10 from the external environment, resist external moisture, solvents and impact, make the chip electrically insulated from the external environment, and enable the overall packaging to exhibit a good sealing performance, and resistance to thermal shock, mechanical vibration and thermal diffusion, etc.

An open structure refers to a structure in which partial or all sequencing region on the electrode layer 102 is optionally exposed after the curing of the packaging body 200 used for packaging the chip body 10, to allow the sequencing region to contact with a liquid to be detected or reagents outside the chip.

A microfluidic structure refers to a structure that precisely controls and manipulates microfluidics through the electrode layer 102. Such a structure may control and direct the fluid to the sequencing region through microfluidic channels, thereby achieving contact between the fluid and the sequencing region.

The example of the present disclosure provides an open packaging structure 300 exemplified in FIG. 8 and FIG. 9.

The packaging body 200 may be a waterproof closed structure, presenting a semi-enclosed state, and comprises a material selected from conventional semiconductor packaging materials compatible with nucleic acid loading and sequencing reagents. The packaging body 200 wraps the chip body 10 for protection to complete the packaging, with the sequencing region formed by the electrode layer 102 being exposed. The circuit pins 201 are drawn out in the corresponding chip bodies 10, and the electrical signals are led out from the chip bodies 10 to the pins to realize the signal transmission.

### [Method For Manufacturing Gene Sequencing Chips]

Further, an example of the present disclosure provides a method for manufacturing a gene sequencing chip, comprising steps of:
S1, providing at least one substrate layer;
S2, disposing at least one electrode layer and at least one protective layer on the substrate layer with the protective layer and the electrode layer being not on the same plane; and
S3, etching on the electrode layer or windowing the protective layer, so that at least a portion of the electrode layer protrudes from the protective layer to form a plurality of electrode parts presenting an array arrangement.

It may be understood that, according to the structural features of the above examples, steps S1~S3 provided in the present disclosure may be realized by only applying conventional semiconductor manufacturing processes and conventional semiconductor materials, such as, without special materials and processes such as deposition, photolithography, etching, etc., and without special silicon wafer substrates. The substrate layer may be a quartz wafer, a glass wafer, a silicon-based wafer or even an integrated circuit wafer. The process has been well-established and has high yield, and may reduce costs and increase process reliability and yield in the manufacturing process.

In step S3, the electrode layer may have an array arrangement as any structure of the above Examples 1-4 for the purpose of arraying the electrode layer on the surface of the substrate layer.

In the arrangements, the electrode layer is composed of a transition metal oxide comprising at least one of titanium dioxide, zirconium dioxide, tantalum pentoxide, niobium hexoxide and hafnium dioxide.

Corresponding to the above Example 1 in the "[Chip Structure]" section, step S2 comprises:
S21, disposing the protective layer on the substrate layer, and
S22, disposing the electrode layer on the protective layer;
and step S3 comprises:
   S31, etching on the electrode layer to form a plurality of electrode parts protruding from the protective layer on the electrode layer.

Corresponding to the above Example 2 in the "[Chip Structure]" section, step S2 comprises:
S21', disposing a first protective layer on the substrate layer,
S22', disposing the electrode layer on the first protective layer, and
S23', disposing a second protective layer on the electrode layer;
and step S3 comprises:
   S31', windowing the second protective layer to form a plurality of second openings presenting an array arrangement and exposing the corresponding portions of the electrode layer to the corresponding second openings, so as to form a plurality of electrode parts on the electrode layer.

Corresponding to the above Example 3 in the "[Chip Structure]" section, step S2 comprises:
step S2 comprises:
   S21", disposing the electrode layer on the substrate layer, and
   S21", disposing the protective layer on the electrode layer;
and step S3 comprises:
   S31", windowing the protective layer to form a plurality of first openings presenting an array arrangement and exposing the corresponding portions of the electrode layer to the corresponding first openings, so as to form a plurality of electrode parts on the electrode layer.

Corresponding to the above Example 4 in the "[Chip Structure]" section, the method for manufacturing the gene sequencing chip further comprises:
S4, stacking and packaging the two gene sequencing chips with the sides provided with electrode layers being opposite.

### [System For Gene Sequencing Chips]

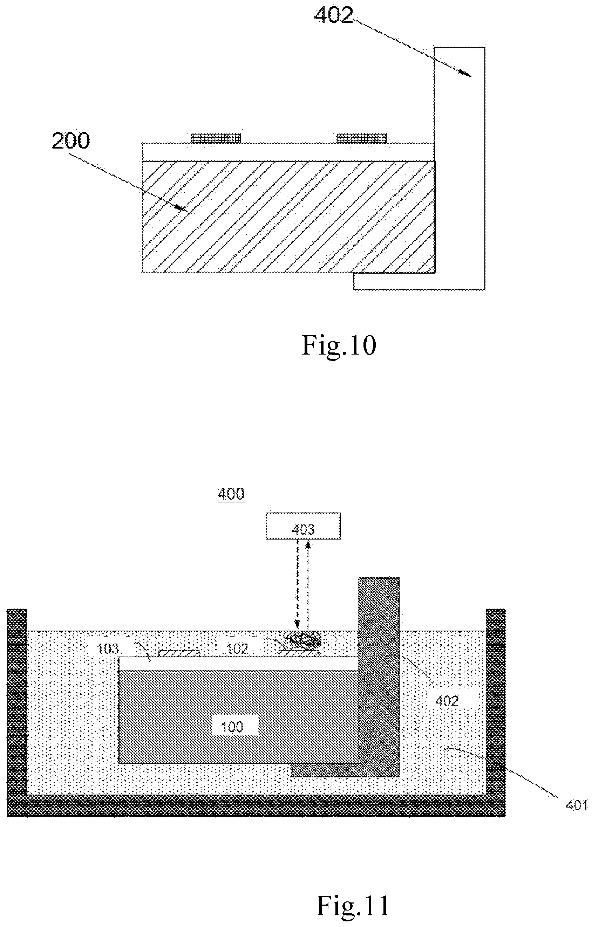

With reference to FIG. 10 and FIG. 11, FIG. 10 illustrates a cross-sectional view of a gene sequencing chip 100 and a gripping mechanism 402 in a fixed state provided in an example of the present disclosure; and FIG. 11 illustrates a structural schematic diagram of a system 400 for gene sequencing provided in an example of the present disclosure. The system 400 comprises:
the gene sequencing chip 100 as described above;
a plurality of test reagent containers 401, provided with a plurality of reaction reagents;
a gripping mechanism 402, for picking up the gene sequencing chip 100 and putting it into the test reagent containers 401 for reactions; and
a signal acquisition mechanism 403, for acquiring signals fed back from the gene sequencing chip 100 to realize sequencing.

Further, the test reagent container is provided with reagent chambers for holding different reaction reagents.

In the example of FIG. 10, the gene sequencing chip 100 formed in Example 1 is assembled with the gripping mechanism 402, wherein the gripping mechanism 402 comprises a support portion 402a below the gene sequencing chip 100, and a handle portion 402b connected to the support portion 402a and protruding above the gene sequencing chip.

It may be understood that the gene sequencing chip 100 has the electrode layer 102 upwardly facing the opening of the test reagent container 401 when put into the test reagent container 401, and the orientation of the support portion 402a may allow the electrode layer 102 disposed on the gene sequencing chip 100 to be completely immersed in the reagent, and makes it more convenient for the above signal acquisition mechanism 403 to acquire electrical signals.

The handle portion 402b may be connected to an external mobile mechanism so that it can drive the gene sequencing chip 100 to move. By fixing the gripping mechanism 402 with a single chip, the handle structure may be used to perform operations such as gripping and transferring the sequencing chip, so as to carry out the loading and sequencing of DNBs.

In an example of the present disclosure, the gripping mechanism 402 has an L-shaped handle structure, which is only schematically showed in the drawings. Those skilled in the art should recognise that any handle structure capable of achieving the above functions is included in the present disclosure. The quantity of gripping mechanisms 402 is not limited in the present disclosure, and a plurality of gripping mechanisms 402 may be packaged with a single chip.

Further, the gripping mechanism 402 may be composed of a material selected from plastics or metals that are compatible with the DNB loading and sequencing reagents, low-cost, easy to process, and not susceptible to aging and wear, such as polymer plastics including but not limited to poly(ether-ether-ketone), polycarbonate, polymethylmethacrylate, or metals such as aluminum alloy, stainless steel and the like. The single gene sequencing chip 100 and the gripping mechanism 402 may be bonded together with a solid or liquid type adhesive, and any adhesive compatible with the DNB loading and sequencing reagents may be used in the present disclosure.

As shown in FIG. 11, the assembled sequencing chip shown in FIG. 10 is immersed into a test reagent container 401 holding a reagent that may be any reagent for processes of chip surface modification, DNB loading and sequencing. In one process of surface modification, DNB loading and sequencing, there may be a plurality of test reagent containers 401 with different reagents inside each, and the gripping mechanism 402 may be used to switch the sequencing chip in different containers and reagents to perform different reactions.

Further, after loading DNBs on the region containing DNB binding sites (a transition metal oxide layer with a spot pattern) on the sequencing chip, optical signals with different wavelengths and energies emitted from the DNBs may be captured by the signal acquisition mechanism 403, which optionally comprises an excitation light source and a camera, for performing the sequencing operation.

It should be noted that if the gene sequencing chip 100 is an integrated circuit chip capable of self-acquiring optical signals, a bioluminescence reaction can be applied by changing biochemical reagents in the test reagent containers 401 to achieve the process of acquiring optical signals for sequencing without an excitation light source and a camera.

### [Method For Cleaning Gene Sequencing Chips]

### Example 1

Further, an example of the present disclosure provides a method for cleaning a gene sequencing chip 100 provided in the above examples, comprising:
S51, allowing a first cleaning solution to flow through the gene sequencing chip or placing the gene sequencing chip in a first cleaning solution for a first predetermined period;
S52, cleaning the chip surface repeatedly with deionized water; and
S53, placing the gene sequencing chip in a stream of inert gas for complete drying.

In particular, following the completion of the gene sequencing process, at least the surface of the gene sequencing chip 100 was treated by allowing a first cleaning solution flow through or was immersed in a first cleaning solution. The first cleaning solution was a 50 mM potassium hydroxide solution containing Triton (a non-ionic surfactant). The gene sequencing chip 100 was immersed in the solution for 10~15 minutes, was placed in a cleaning device, where the chip surface was repeatedly cleaned with deionized water for more than 3 times, and was placed in a drying device with flowing nitrogen gas for complete drying.

### Example 2

In an alternative example of the present disclosure, the method for cleaning the gene sequencing chip 100 comprises:
S51', allowing a second cleaning solution heated to a first predetermined temperature flow through the gene sequencing chip or placing the gene sequencing chip in a second cleaning solution heated to a first predetermined temperature for a second predetermined period;
S52', cleaning the chip surface repeatedly with deionized water; and
S53', placing the gene sequencing chip in a stream of inert gas for complete drying.

In particular, following the completion of the gene sequencing process, the gene sequencing chip 100 was treated by allowing a second cleaning solution heated to 80°C to flow through or was placed in a second cleaning solution heated to 80°C for 5~10 minutes, wherein the second cleaning solution was a mixture of ammonia water and hydrogen peroxide in a predetermined ratio. Thereafter, the gene sequencing chip 100 was placed in a cleaning device, where it was repeatedly cleaned with deionized water for more than 3 times and was placed in a drying device with flowing nitrogen gas for complete drying.

### Example 3

S51", pre-treating the gene sequencing chip to expose the gene sequencing chip;
S52'', placing the chip in a plasma atmosphere for a third predetermined period and cleaning the chip surface repeatedly with deionized water; and
S53'', placing the gene sequencing chip in a stream of inert gas for complete drying.

In particular, following the completion of the gene sequencing process, the gene sequencing chip 100 was pre-treated for exposure. The chip was placed in a plasma atmosphere (e.g., an argon atmosphere) created by a plasma device for 30 minutes. Thereafter, the gene sequencing chip was placed in a cleaning device, where it was cleaned with deionized water to remove dust particles, and was placed in a drying device with flowing nitrogen gas for complete drying.

### Example 4

S51‴, heating and sonication of the gene sequencing chip directly exposed to isopropanol for a fourth predetermined period, and cleaning the chip surface repeatedly with deionized water;
S52‴, rinsing or immersing the gene sequencing chip with sodium dodecyl sulfate solution, and cleaning the chip surface again repeatedly with deionized water; and
S53‴, placing the gene sequencing chip in a stream of inert gas for complete drying.

In particular, following the completion of the gene sequencing process, the surface of the gene sequencing chip 100 was directly exposed to IPA (Isopropyl Alcohol) for heating and sonication for 15 min, and then the chip was placed in a cleaning device, where the chip surface was repeatedly rinsed with deionized water. Thereafter, the chip surface was rinsed or immersed with SDS (Sodium Dodecyl Sulfate) solution, then was placed in a cleaning device, where the chip surface was again rinsed with deionized water, and was placed in a drying device with flowing nitrogen gas for complete drying.

### [Comparative Examples]

The examples of the present disclosure further comprise comparative examples for validating the nucleic acid microarray and biochemical performance on the chip surfaces after cleaning.
1. The optical signal graph of the gene sequencing chip 100 at the first loading of DNBs was used as a comparative example; and the optical signal graph of the gene sequencing chip 100 measured after 6 repeated cleaning cycles and the reloading of DNBs was used as an experimental reference.

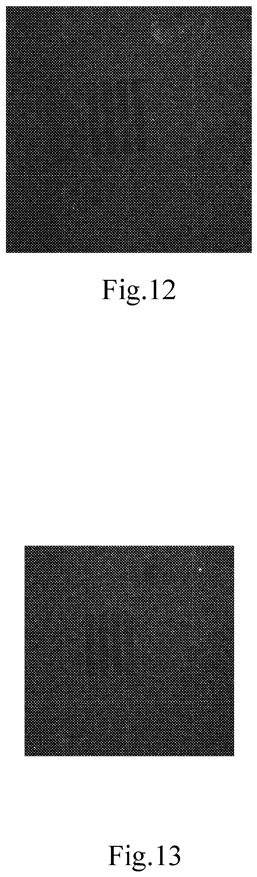

With reference to FIG. 12 to FIG. 13, FIG. 12 illustrates an optical signal graph of a gene sequencing chip provided in an example of the present disclosure for the first loading of DNBs, and FIG. 13 illustrates an optical signal graph of a gene sequencing chip provided in an example of the present disclosure after 6 times of cleanings for the loading of DNBs.

From the comparison of FIG. 12 and FIG. 13, it can be seen that there was no significant difference in the specific adsorption of nucleic acid microarrays on the chip surface between being not cleaned and after 6 repeat cleanings, and both of them performed well (loading rate > 90%).

2. The sequencing quality of the gene sequencing chip at cycle 1, cycle 6 and cycle 150 was recorded and compared.

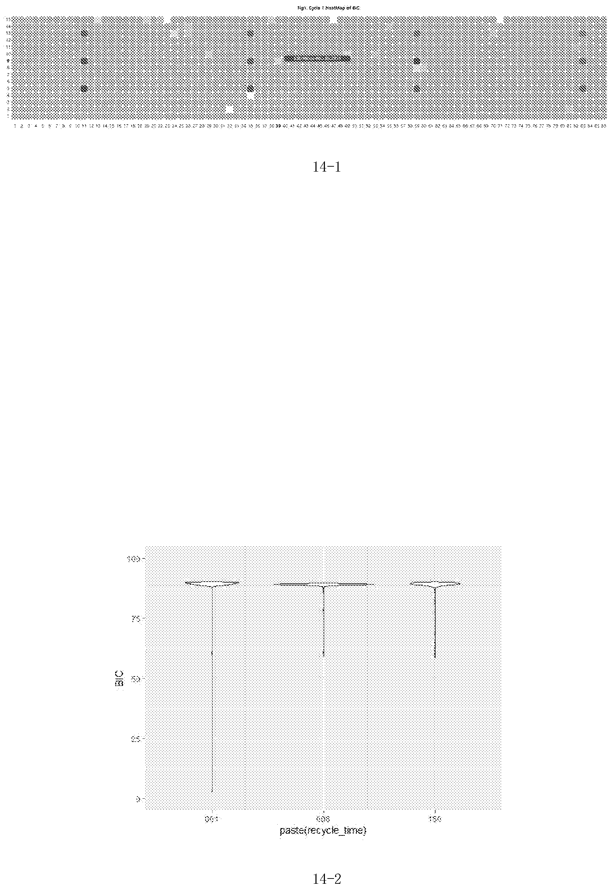

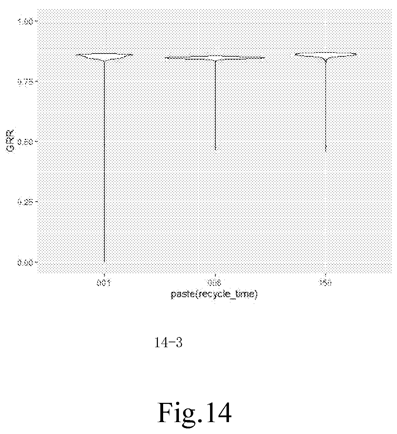

With reference to FIG. 14, FIG. 14 illustrates comparison of sequencing quality of a gene sequencing chip provided in an example of the present disclosure at cycle 1, cycle 6 and cycle 150.

As can be seen in FIG. 14, there was no significant difference among the sequencing quality at cycle 6, cycle 150 and cycle 1 after repeated cleanings of the chip, proving that the integrated sequencing chip had good stability.

3. The performance of gene sequencing chips before and after immersion in different sequencing reagents for five days was compared.

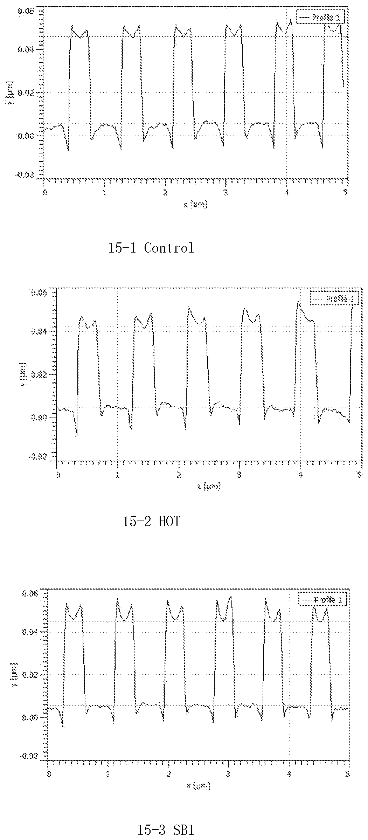

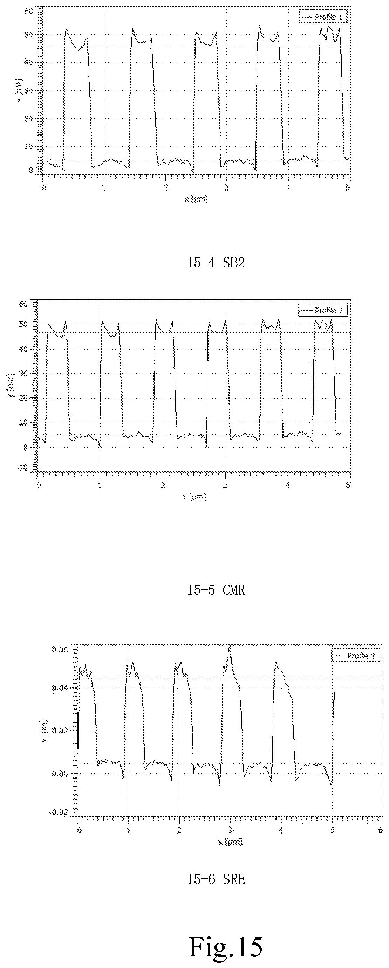

FIG. 15 illustrates AFM plots of the gene sequencing chips in an example of the present disclosure before and after immersion in different sequencing reagents for 5 days.

FIG. 15 illustrates the comparison of the peak height measurements between the chips after immersion in various reagents for 5 days and a control chip, which shows that the electrode layer was not corroded when immersed in several common sequencing reagents for a long period.

### [Method of Using Gene Sequencing Chips]

### Example 5

An example of the present disclosure provides a sequencing method of using the gene sequencing chips of the present disclosure.

Sequencing instrument: MGI DNBSEQ-E25RS.

Sequencing reagents: MGI DNBSEQ-E25RS High-Throughput Sequencing Reagent Kit SE100 with the Cat. No. of 940-000263-00.

The sequencing procedures on a sequencer were as follows:
1. preparing DNBs according to the manufacturer's instructions, as can be seen in MGI High-Throughput Sequencing Reagent Kit (DNBSEQ-E25RS FCL SE100) with the Cat. No. of 940-000263-00;
2. preparing a sequencing reagent reservoir by steps of: thawing, checking for integrity, addition of first signaling factor, second signaling factor and buffers, and mixing thoroughly;
3. placing the gene sequencing chip of the present disclosure in the sequencer;
4. placing the sequencing reagent reservoir and liquid waste container in the sequencer;
5. loading the DNBs onto the gene sequencing chip; and
6. sequencing steps: filling in the information as instructed followed by sequencing;
8. the patent publication CN113748216A could be referred to for the sequencing step; and patent application CN201880056178 could be referred to for the microfluidic chip for fluid communication.

### [Comparison between the first loading and the reloading after sequencing of a non-reusable chip]

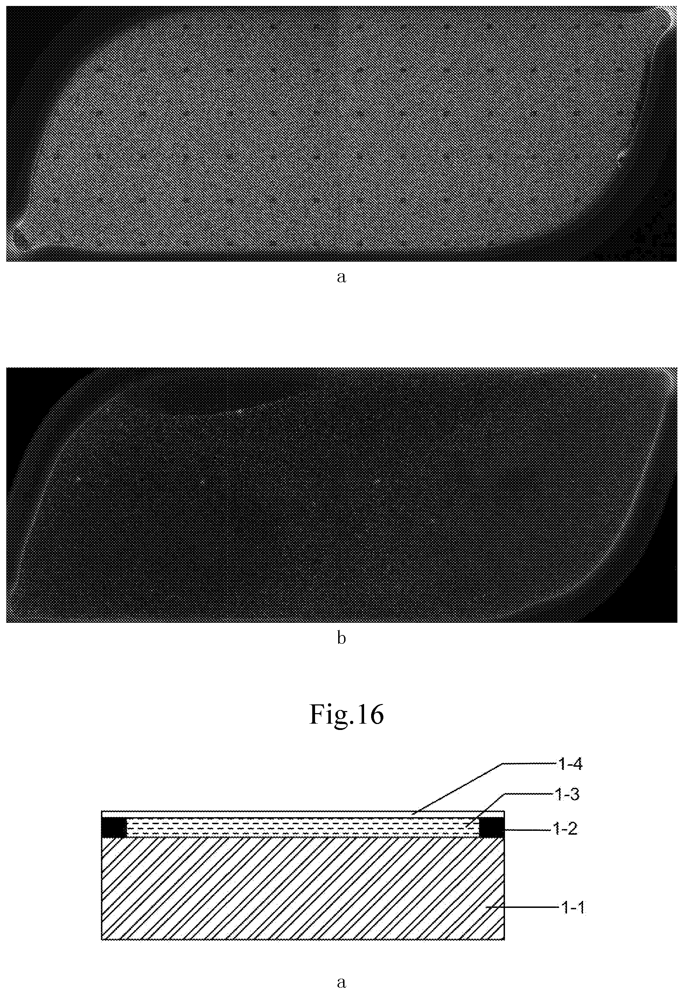

A comparison of the signal graphs between the first use and the reuse after sequencing of a non-reusable chip is shown in FIG. 16.

The sequencing signals in the first use are shown in FIG. 16a, wherein the luminescent and non-luminescent points had clear distribution, and the DNB luminescence at each pixel point could be clearly identified. After complete loading, the DNBs were evenly loaded in the loading area in the field of vision. The test area had a clearly visible pattern, indicating less non-specific adsorption and stable chip surface properties. The sequencing signals in the reuse after sequencing are shown in FIG. 6b, wherein the DNBs on the chip were thinly loaded, and had such fuzzy luminescence that the luminescent points were almost indistinguishable from the non-luminescent points. The pattern of the test area was not recognizable.

### [Measurement of the efficiency of reloading DNBs on a gene sequencing chip of the present disclosure]

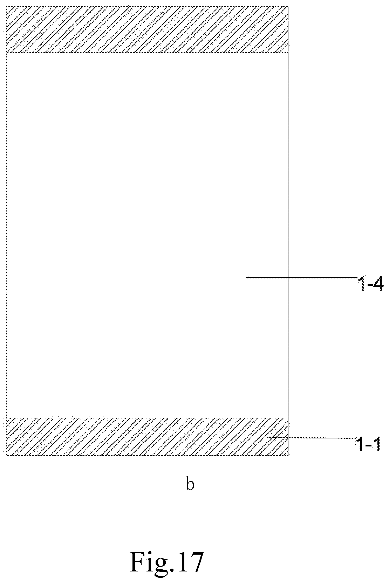

DNBs were manually loaded on a reusable gene sequencing chip of the present disclosure and the DNB loading results were observed. A cross-sectional schematic of the manual loading device is shown in FIG. 17a, and a top view of the manual loading device is shown in FIG. 17b. Reference signs in FIG. 17 are as follows: 1-1, a silicon-based chip; 1-2, a padding device, which may be a 3M adhesive or a rubber strip with an appropriate height; 1-3, a flowing reagent and a cavity thereof; and 1-4, an upper cover plate, which may be a COP sheet, a coverslip, or any applicable material. The manual loading device was configured with a fluid cavity with openings at the top and bottom above the chip, so that various reagents can flow into from the top and flow out from the bottom, ensuring that various reagents flow through the liquid cavity as required. Manual loading procedure comprises:
a) building the loading device (as shown in FIG. 17a);
b) mixing DNBs with an appropriate DNB loading buffer, injecting into the cavity through the opening at the chip top, and then incubating for 30 min in an airtight environment;
c) injecting an appropriate amount of DNB wash buffer (DRB), DNB collision buffer (DCB), protein wash buffer (PWB), DNB collision buffer (DCB), and CPAS cleaning reagent (WB1) in order to make the DNBs fixed on the chip, wherein the reagents used above and reagents in the kit with the same Cat. No. as above were provided by MGI;
d) adding appropriate fluorescent probe primers (fluorescent groups including but not limited to cy3, and cy5) into the cavity and incubating for 20 minutes at room temperature away from light, so that the fluorescent probes were attached to DNBs at each site, wherein the probe primers were customized from Shanghai Sangon; and
e) observing the DNB luminescence at each site under a fluorescence microscope to determine the loading effect and the degree of non-specific adsorption.

In summary, with the structural features of the electrode layer 102 and the protective layer 103 as proposed in the general inventive concept, the present disclosure has the following advantages over the existing designs employing molecular layer modifications:
1. The chip body 10 may be realized by applying conventional semiconductor manufacturing processes and conventional semiconductor materials, without special materials and processes, such as special silicon wafer substrates. The process has been well-established and has high yield, and can reduce the cost during the manufacturing.
2. With a protective layer 103, the arrayed sites formed on the electrode layer 102 are resistant to the corrosion of biochemical reagents, and the structure is more suitable for DNA adsorption and binding. In addition, the chip may still possess the original characteristics in terms of the morphology and material after some times of reuse, and thus may be reused.
3. In the prior art, the monomolecular layer modification is not resistant to high temperature, sensitive to pH and special components of the reagents, unstable in performance, prone to failure of the specific adsorption layer in a variety of common conditions and even after long storage, and prone to physical contact damage (such as surface rubbing, etc.), all of which may result in a decrease in the output throughput. The chip body 10 is more reliable compared to those in the prior art.
4. In the prior art, a chip has a molecular modification layer and a physically planar surface, the nucleic acids adsorbed on the surface are easily washed away in the high-speed flow of reagents, resulting in interrupted sequencing. In contrast, the chip body 10 provided in the embodiment of the present disclosure has a protruding surface, which may better retain the nucleic acids and improve the stability of sequencing.

Those skilled in the art should understand that all or part of the sub-modules involved in a gene sequencing chip 100, a packaging structure 300, and a system 400 provided in the examples of the present disclosure may be combined and replaced by means of combination, simple changes, and mutual transformations, such as changing the position of each component, or integrating the product composed of these components or a detachable design in the product. As long as the combined components may constitute a device/apparatus/system with a specific function, replacing the corresponding components of the present disclosure with such a device/apparatus/system also falls within the protection scope of the present disclosure.

**In** the present description, the description that refers to the terms "an embodiment", "some embodiments", "examples", "specific examples", or "some examples" means that the specific features, structures, materials, or characteristics described in conjunction with the embodiment or example are included in at least one embodiment or example of the present disclosure. In the present description, illustrative expressions of the above terms are not necessarily directed to the same embodiment or example. Moreover, the specific features, structures, materials or characteristics described may be combined in a suitable manner in any one or more embodiments or examples. Furthermore, without contradicting each other, those skilled in the art may incorporate and combine different embodiments or examples and features of different embodiments or examples described in the present disclosure.

Although the examples of the present disclosure have been shown and described above, it is understood that the above examples are exemplary and are not to be construed as a limitation of the present disclosure. A person having ordinary skill in the art may perform changes, modifications, replacements and variations of the above examples within the scope of the present disclosure.

## Claims

1. A gene sequencing chip comprising at least one chip body, the chip body comprising:
a substrate layer, provided with a circuit;
at least one electrode layer on the substrate layer; and
at least one protective layer on the substrate layer, for insulation of the substrate layer from the outside;
wherein, the protective layer and the electrode layer are not located on the same plane, at least a portion of the electrode layer protrudes from the protective layer, and the portion of the electrode layer protruding from the protective layer comprises a plurality of electrode parts presenting an array arrangement and loaded with DNBs to form a sequencing region.

2. The gene sequencing chip according to claim 1, wherein
the electrode layer is formed with a transition metal oxide;
wherein the transition metal oxide comprises at least one of titanium dioxide, zirconium dioxide, tantalum pentoxide, niobium hexoxide and hafnium dioxide.

3. The gene sequencing chip according to claim 1 or 2, wherein
the protective layer is disposed on the substrate layer; and
the electrode layer fully protrudes from the protective layer, and the electrode layer has a plurality of electrode parts presenting an array arrangement disposed on the protective layer.

4. The gene sequencing chip according to claim 1 or 2, wherein
the protective layer comprises a first protective layer and a second protective layer, and the first protective layer, the electrode layer and the second protective layer are sequentially laminated on the substrate layer;
the electrode layer is disposed on the first protective layer; and
the second protective layer is provided with a plurality of second openings presenting an array arrangement and exposing the corresponding portions of the electrode layer to the corresponding second openings, to form a plurality of electrode parts on the electrode layer.

5. The gene sequencing chip according to claim 1 or 2, wherein
the electrode layer is disposed on the substrate layer; and
the protective layer is disposed on the electrode layer and provided with a plurality of first openings presenting an array arrangement and exposing the corresponding portions of the electrode layer to the corresponding first openings, to form a plurality of electrode parts on the electrode layer.

6. The gene sequencing chip according to claim 1, the gene sequencing chip comprises two chip bodies connected by splicing along the lengthwise direction.

7. The gene sequencing chip according to any one of claims 3-5, wherein there are two gene sequencing chips, and the two gene sequencing chips are stacked along the thickness direction, and arranged with the sides provided with the electrode layers being opposite.

8. The gene sequencing chip according to claims 1-7, wherein the electrode parts are loaded with DNBs to form a sequencing region with arrayed sites.

9. A packaging structure for a gene sequencing chip, comprising:
the gene sequencing chip of any one of claims 1-8; and
a packaging body, encasing the gene sequencing chip to form an open structure or a microfluidic structure.

10. A method for manufacturing a gene sequencing chip, comprising steps of:
S1, providing at least one substrate layer;
S2, disposing at least one electrode layer and at least one protective layer on the substrate layer with the protective layer and the electrode layer being not on the same plane; and
S3, etching on the electrode layer or windowing the protective layer, so that at least a portion of the electrode layer protrudes from the protective layer to form a plurality of electrode parts presenting an array arrangement.

11. The method according to claim 10, wherein the electrode layer is composed of a transition metal oxide comprising at least one of titanium dioxide, zirconium dioxide, tantalum pentoxide, niobium hexaoxide and hafnium dioxide.

12. The method according to claim 10, wherein
step S2 comprises:
disposing the protective layer on the substrate layer; and
disposing the electrode layer on the protective layer; and
step S3 comprises:
etching on the electrode layer to form a plurality of electrode parts protruding from the protective layer on the electrode layer.

13. The method according to claim 10, wherein
step S2 comprises:
disposing a first protective layer on the substrate layer;
disposing the electrode layer on the first protective layer; and
disposing a second protective layer on the electrode layer; and
step S3 comprises:
windowing the second protective layer to form a plurality of second openings presenting an array arrangement and exposing the corresponding portions of the electrode layer to the corresponding second openings, so as to form a plurality of electrode parts on the electrode layer.

14. The method according to claim 10, wherein
step S2 comprises:
disposing the electrode layer on the substrate layer; and
disposing the protective layer on the electrode layer; and
step S3 comprises:
windowing the protective layer to form a plurality of first openings presenting an array arrangement and exposing the corresponding portions of the electrode layer to the corresponding first openings, so as to form a plurality of electrode parts on the electrode layer.

15. The method according to any one of claims 10-14, wherein there are two gene sequencing chips, and the method further comprises:
S4, stacking and packaging the two gene sequencing chips with the sides provided with electrode layers being opposite.

16. A system for a gene sequencing chip, comprising:
the gene sequencing chip of claims 1-8;
a plurality of test reagent containers, for holding different reaction reagents;
a gripping mechanism, for picking up the gene sequencing chip and putting it into the test reagent containers for reactions; and
a signal acquisition mechanism, for acquiring signals from the gene sequencing chip to realize sequencing.

17. The system according to claim 16, wherein the gripping mechanism comprises a support portion below the gene sequencing chip, and a handle portion connected to the support portion and protruding above the gene sequencing chip.

18. The system according to claim 17, wherein the gripping mechanism has an L-shaped handle structure.

19. A method for cleaning the gene sequencing chip of any one of claims 1-8, comprising:
allowing a first cleaning solution to flow through the gene sequencing chip or placing the gene sequencing chip in a first cleaning solution for a first predetermined period;
cleaning the chip surface repeatedly with deionized water; and
placing the gene sequencing chip in a stream of inert gas for complete drying;
wherein the first cleaning solution is a potassium hydroxide solution containing a nonionic surfactant.

20. A method for cleaning the gene sequencing chip of any one of claims 1-8, comprising:
allowing a second cleaning solution heated to a first predetermined temperature flow through the gene sequencing chip or placing the gene sequencing chip in a second cleaning solution heated to a first predetermined temperature for a second predetermined period;
cleaning the chip surface repeatedly with deionized water; and
placing the gene sequencing chip in a stream of inert gas for complete drying;
wherein the second cleaning solution is a mixture of ammonia water and hydrogen peroxide in a predetermined ratio.

21. A method for cleaning the gene sequencing chip of any one of claims 1-8, comprising:
pre-treating the gene sequencing chip to expose the gene sequencing chip;
placing the chip in a plasma atmosphere for a third predetermined period and cleaning the chip surface repeatedly with deionized water; and
placing the gene sequencing chip in a stream of inert gas for complete drying.

22. A method for cleaning the gene sequencing chip of any one of claims 1-8, comprising:
heating and sonication of the gene sequencing chip directly exposed to isopropanol for a fourth predetermined period, and cleaning the chip surface repeatedly with deionized water;
rinsing or immersing the gene sequencing chip with sodium dodecyl sulfate solution, and cleaning the chip surface again repeatedly with deionized water; and
placing the gene sequencing chip in a stream of inert gas for complete drying.

23. A method for sequencing, comprising:
preparing DNBs;
loading the DNBs onto the gene sequencing chip of any one of claims 1-8; and
sequencing the DNBs loaded onto the gene sequencing chip.

24. The system according to claim 23, wherein the gene sequencing chip is a gene sequencing chip capable of being reused after cleaning.

25. The system according to claim 24, wherein the cleaning is performed using the method of any one of claims 19-22.

26. The system according to any one of claims 23-25, wherein the sequencing is automated sequencing or manual sequencing.

27. The system according to any one of claims 23-25, wherein the efficiency of loading the DNBs is determined.
